# EUROPÄISCHE PATENTSCHRIFT

(11) **EP 3 468 482 B1**
(45) Veröffentlichungstag und Bekanntmachung des Hinweises auf die Patenterteilung: **26.10.2022**
(21) Anmeldenummer: 17730766.7
(22) Anmeldetag: 14.06.2017
(51) Int. Cl.: A61B 17/02

(54) **VORRICHTUNG ZUR VERMINDERUNG DER RETRAKTION EINER FASZIE ODER EINES WEICHTEILMANTELS BEI EINEM OFFENEN WEICHTEILDEFEKT**
DEVICE FOR REDUCING THE RETRACTION OF A FASCIA OR OF A SOFT-TISSUE MANTLE IN AN OPEN SOFT-TISSUE DEFECT
DISPOSITIF POUR DIMINUER LA RÉTRACTION D'UN FASCIA OU D'UNE ENVELOPPE DE TISSU MOU LORS D'UN DÉFAUT OUVERT D'UN TISSU MOU

(30) Priorität: 14.06.2016 DE 102016210574
(43) Veröffentlichungstag der Anmeldung: 17.04.2019
(73) Patentinhaber: Fasciotens GmbH, 45138 Essen (DE)
(72) Erfinder: LILL, Gereon, 50858 Köln (DE)
(74) Vertreter: Kutzenberger Wolff & Partner
(86) Internationale Anmeldenummer: PCT/EP2017/064546
(87) Internationale Veröffentlichungsnummer: WO 2017/216224

(56) Entgegenhaltungen:
- WO-A1-2015/155176
- US-B1- 6 354 994

## Beschreibung

### Gebiet der Erfindung

Die Erfindung betrifft eine Vorrichtung zur Verminderung der Retraktion der Ränder einer geöffneten Faszie eines Patienten mit einem offenen Weichteildefekt, wobei die Vorrichtung ein Halteelement, welches in einem ersten Abstand von dem Patienten gegenüber dem Patienten festlegbar ist, und ein Anbindungselement umfasst, welches eine Vielzahl von Anbindungspunkten aufweist, die zur Anbindung von Zugmitteln eingerichtet sind, die mit einem Rand der Faszie verbindbar sind, wobei das Anbindungselement mit dem Halteelement derart verbunden ist, dass es in einem zweiten Abstand zu dem Patienten angeordnet ist, welcher kleiner ist als der erste Abstand.

Die Erfindung betrifft ferner ein Kit zur Behandlung eines offenen Weichteildefekts mit mindestens einem Schwamm zum Ausfüllen des Weichteildefekts.

Die Erfindung kann bei offenen Weichteildefekten und/oder offenen Wunden Anwendung finden. Solche Weichteildefekte bzw. Wunden können beispielsweise als Bauchwanddefekt, Rückenwanddefekt, Weichteildefekt im Bereich der Hüfte oder Weichteildefekt an den Gliedmaßen vorliegen.

### Stand der Technik

In der US 6 354 994 B1 wird ein chirurgischer Retraktor beschrieben, der zur Retraktion der Rippen sowie der Thoraxregion eines Patienten eingesetzt werden kann. Der Retraktor umfasst eine Hakenplatte, an der mehrere Haken angeordnet sind, die beispielsweise an einem Brustbein eines Patienten angebunden werden können. Die Hakenplatte ist über ein Seil mit einer Halterung verbunden, an der ein Ratschenmechanismus vorgesehen ist, mit welchem eine Zugkraft über das Seil, die Hakenplatte und die Haken auf das Brustbein des Patienten aufgebracht werden kann.

Eine Vorrichtung zur Verminderung der Retraktion der Ränder einer geöffneten Faszie eines Patienten mit einem offenen Weichteildefekt ist aus der WO 2015/155176 A1 bekannt. Mittels dieser Vorrichtung kann eine Zugkraft auf die Ränder der geöffneten Faszie aufgebracht werden. Die Ränder der geöffneten Faszie werden auf Abstand gehalten, so dass der Zugang zu dem unter dem Weichteildefekt liegenden Körperinnenraum, z. B. dem Bauchraum, möglich ist, beispielsweise um eine Unterdruck-Wundtheraphie durchzuführen. Durch die Zugkraft wird die Spannung der Faszie aufrechterhalten und damit die Retraktion der Ränder der Faszie vermindert. Die Ränder der Faszie können nach Abschluss der Therapie aneinander angelegt und direkt verschlossen werden.

Zur Durchführung einer Unterdruck-Wundtherapie ist es erforderlich, den offenen Weichteildefekt bzw. die offene Wunde abzudichten, so dass ein Unterdruck erzeugt werden kann. Zur Abdichtung können beispielsweise gasdichte Folien verwendet werden, welche einen gegenüber der Umgebung abgedichteten Unterdruckbereich definieren. Die bekannte Vorrichtung weist ein Halteelement auf, welches in einem ersten Abstand von dem Patienten gegenüber dem Patienten festlegbar ist. Das Halteelement ist über mehrere Seile mit einem Anbindungselement verbunden, welches eine Vielzahl von Anbindungspunkten aufweist, die zur Anbindung von Zugmitteln eingerichtet sind. Die Zugmittel werden mit einem Rand der Faszie verbunden, so dass eine Zugkraft auf die Faszie wirkt. Das Anbindungselement ist in einem zweiten Abstand zu dem Patienten angeordnet, welcher kleiner ist als der erste Abstand. Bei der Durchführung einer Vakuum-Wundtherapie wird die Vorrichtung typischerweise innerhalb des Unterdruckbereichs angeordnet. Um ein Nachspannen der Zugmittel bzw. der Seile auch bei angelegtem Unterdruck zu ermöglichen, ist an dem Halteelement ein Betätigungselement vorgesehen, welches außerhalb des abgedichteten Bereichs angeordnet wird. Um die Unterdruck-Wundtherapie zu ermöglichen, ist das Halteelement mit einem abgedichteten Gehäuse ausgebildet und an mehreren Stellen gegenüber der gasdichten Folie angedichtet. Zur Abdichtung des Unterdruckbereichs ist daher eine erhebliche Anzahl an Handgriffen erforderlich.

### Offenbarung der Erfindung

Vor diesem Hintergrund liegt der Erfindung die Aufgabe zugrunde, eine Abdichtung des Unterdruckbereichs mit möglichst wenigen Handgriffen zu ermöglichen.

Die Aufgabe wird gelöst durch eine Vorrichtung zur Verminderung der Retraktion der Ränder einer geöffneten Faszie eines Patienten mit einem offenen Weichteildefekt, wobei die Vorrichtung ein Halteelement, welches in einem ersten Abstand von dem Patienten gegenüber dem Patienten festlegbar ist, und ein Anbindungselement umfasst, welches eine Vielzahl von Anbindungspunkten aufweist, die zur Anbindung von Zugmitteln eingerichtet sind, die mit einem Rand der Faszie verbindbar sind, wobei das Anbindungselement mit dem Halteelement derart verbunden ist, dass es in einem zweiten Abstand zu dem Patienten angeordnet ist, welcher kleiner ist als der erste Abstand, wobei das Anbindungselement mit dem Halteelement über genau ein Zugkraftübertragungselement verbunden ist, wobei die Vorrichtung einen Zugkraftmesser zur Messung der über das Zugkraftübertragungselement übertragenen Zugkraft aufweist.

Erfindungsgemäß ist das Anbindungselement über genau ein Zugkraftübertragungselement mit dem Halteelement verbunden, welches die gesamte Zugkraft von dem Anbindungselement auf das Halteelement überträgt. Bei der Durchführung einer Unterdruck-Wundtherapie kann das Halteelement außerhalb des Unterdruckbereichs angeordnet werden und das innerhalb des Unterdruckbereichs angeordnete Anbindungselement halten. Da lediglich eine Verbindung zwischen dem Halteelement und dem Anbindungselement besteht, wird die Abdichtung des Unterdruckbereichs erleichtert. Eine den Unterdruckbereich abgrenzende Folie muss nur an einer Stelle gegenüber dem Zugkraftübertragungselement abgedichtet werden. Insofern wird eine Abdichtung des Unterdruckbereichs mit wenigen Handgriffen ermöglicht.

Das Halteelement ist an dem Körper des Patienten festlegbar, so dass sich das Halteelement bei einer Lageänderung des Patienten mit diesem mitbewegen kann. Eine bevorzugte Ausgestaltung sieht vor, dass das Halteelement als Stange ausgebildet ist. Das Halteelement ist bevorzugt parallel zu einer Körperoberfläche des Patienten anordbar. Die Anbindung kann über eine am Körper des Patienten festlegbare Befestigungseinrichtung, insbesondere eine Gurtbefestigung und/oder eine Stütze, erfolgen. Es können mehrere Befestigungseinrichtung vorgesehen sein. Bevorzugt ist eine Ausgestaltung mit zwei Befestigungseinrichtungen, die im oberen (kranialen) und unteren (kaudalen) Bereich des Abdomens oder des Rückens angeordnet werden können. Bevorzugt ist die Befestigungseinrichtung derart ausgebildet, dass sie auf die Haut des Patienten aufgelegt werden kann oder an einem Knochen des Patienten befestigt werden kann. Die Befestigung kann beispielsweise am Brustbein und/oder am vorderen Beckenring erfolgen. Bevorzugt ist die Befestigungseinrichtung derart vorgesehen, dass sie auf die Haut in einem Bereich in der Umgebung des Weichteildefekts auflegbar ist, in welchem durch die Vorrichtung keine Zugkräfte auf die unter der Haut liegende Faszie aufgebracht werden. Eine weitere bevorzugte Ausgestaltung sieht vor, dass die Befestigungseinrichtung ein auf die Haut des Patienten auflegbares Druckverteilungsmittel aufweist. Das Druckverteilungsmittel kann ein Polster aufweisen, insbesondere ein Schaumstoffpolster. Das Druckverteilungsmittel ist bevorzugt mit einem Medium füllbar. Als Medium zum Füllen des Druckverteilungsmittels kann ein Gas, insbesondere Luft, eine Flüssigkeit, insbesondere Wasser, oder ein Gel verwendet werden. Das Druckverteilungsmittel kann eine Kammer aufweisen, in welche das Medium einbringbar ist. Über das Druckverteilungsmittel können auf die Haut des Patienten wirkenden Druckkräfte gleichmäßig verteilt werden, so dass die Gefahr der Bildung von Druckstellen auf der Haut verringert wird. Besonders bevorzugt weist die Befestigungseinrichtung ein Druckverteilungsmittel mit mehreren, insbesondere zwei, Kammern auf. Vorteilhaft ist es, wenn die Kammern derart ausgebildet sind, dass sie wechselweise mit einem Medium gefüllt werden können, so dass die Druckkräfte abwechselnd in verschiedene Bereiche der Haut eingeleitet werden können. Die Kammern sind bevorzugt nach Art von konzentrischen Kreisen ausgebildet, wobei eine erste Kammer als ein Kreisring ausgebildet ist, der eine zweite Kammer umschließt.

Eine bevorzugte Ausgestaltung der Erfindung sieht vor, dass die Vorrichtung mehrere Stützen aufweist, welche das Halteelement jeweils mit einem auf die Haut des Patienten auflegbaren Druckverteilungsmittel verbinden. Hierdurch kann das Halteelement in dem ersten Abstand und dem offenen Weichteildefekt vorgelagert angeordnet werden.

Gemäß einer bevorzugten Ausgestaltung weist die Befestigungseinrichtung ein Implantat, auf, welches an einem Knochen des Patienten befestigbar ist. Das Implantat kann als Fixateur interne oder externe ausgebildet sein. Das Implantat ist besonders bevorzugt ein bereits im Körper des Patienten vorhandenes Implantat, wie beispielsweise ein Wirbelsäulenimplantat. Es ist alternativ möglich, das Implantat zusammen mit der Vorrichtung zur Verminderung der Retraktion der Faszien einzubringen. Das Implantat kann ein oder mehrere Längsträger aufweisen. Die Längsträger des Implantats können an Knochen des Patienten befestigbar sein, beispielsweise an Wirbelkörpern der Wirbelsäule. Zur Befestigung des Implantats, insbesondere der Längsträger, an den Knochen umfasst das Implantat bevorzugt ein oder mehrere Verbindungsmittel, insbesondere eine oder mehrere Schrauben.

Bevorzugt ist es, wenn die Vorrichtung mehrere Stützen aufweist, welche das Halteelement mit einem Implantat oder mehreren Implantaten verbinden, welche an einem Knochen des Patienten festlegbar sind.

Bevorzugt ist das Zugkraftübertragungselement in einer Ausnehmung in dem Halteelement geführt, so dass das Zugkraftübertragungselement zur Einstellung des Abstands zwischen dem Halteelement und dem Anbindungselement bewegbar ist. Insofern kann das Zugkraftübertragungselement in einer quer, insbesondere senkrecht, zur Körperoberfläche orientierten Richtung bewegt werden.

Gemäß einer bevorzugten Ausgestaltung ist an dem Zugkraftübertragungselement ein Dichtelement angeordnet. An dem Dichtelement kann eine Folie zum Abdichten des Unterdruckbereichs angebracht werden. Das Dichtelement kann als Dichtring ausgebildet sein. Bevorzugt ist das Dichtelement entlang des Zugkraftübertragungselements verschiebbar angeordnet, so dass das Zugkraftübertragungselement bewegt, insbesondere gedreht, werden kann, ohne eine an dem Dichtelement angebrachte Abdichtung zu beeinträchtigen. Das Dichtelement kann ein Innengewinde aufweisen, welches mit einem an dem Zugkraftübertragungselement angeordneten Außengewinde in Eingriff steht.

Gemäß einer bevorzugten Ausgestaltung weist das Zugkraftübertragungselement einen Luftkanal auf. Wenn das Halteelement außerhalb des Unterdruckbereichs und das Anbindungselement innerhalb des im Unterdruckbereichs angeordnet ist, kann über den Luftkanal des Zugkraftübertragungselements ein Unterdruck im Unterdruckbereich erzeugt werden. Zur Bildung des Luftkanals kann das Zugkraftübertragungselement hohl ausgebildet sein, beispielsweise als Rohr oder als Schlauch.

Bevorzugt verbindet der Luftkanal eine erste Öffnung zum Anschluss einer Vakuumpumpe mit einer im Bereich des Anbindungselements angeordneten, zweiten Öffnung. An der ersten Öffnung kann ein Anschlussstutzen zum Anschluss der Vakuumpumpe vorgesehen sein. Die erste Öffnung und die zweite Öffnung können beispielsweise an zwei gegenüberliegenden Stirnseiten des Zugkraftübertragungselements angeordnet sein. Bevorzugt weist die Vorrichtung einen Verschluss auf, über welchen der Luftkanal wahlweise abgedichtet werden kann.

Die Erfindung sieht vor, dass die Vorrichtung einen Zugkraftmesser zur Messung der über das Zugkraftübertragungselement übertragenen Zugkraft aufweist. Der Zugkraftmesser ist bevorzugt als Federwaage ausgebildet. Mit der Federwaage kann die Zugkraft anhand der Dehnung einer Druckfeder oder einer Zugfeder, insbesondere einer Schraubendruckfeder oder Schraubenzugfeder, ermittelt werden. Alternativ kann der Zugkraftmesser als induktiver Kraftmesser, kapazitiver Kraftmesser, optischer Kraftmesser, Dehnungsmessstreifen, magnetischen Kraftmesser, elektromagnetischen Kraftmesser oder piezoelektrischen Kraftmesser ausgestaltet sein. Der Zugkraftmesser, insbesondere eine Anzeige des Zugkraftmessers, ist bevorzugt zwischen dem Halteelement und dem Zugkraftübertragungselement angeordnet, so dass die Anzeige des Zugkraftmessers auch dann abgelesen werden kann, wenn das Anbindungselement im abgedichteten Unterdruckbereich angeordnet ist.

Bevorzugt weist der Zugkraftmesser eine Druckfeder auf, welche ein erstes Federende umfasst, das gegenüber dem Halteelement feststehend gelagert ist, und ein zweites Federende, das gegenüber einem Mitnehmer des Zugkraftübertragungselements oder gegenüber einem auf einem Außengewinde des Zugkraftübertragungselements aufsitzenden Befestigungselement, insbesondere einer Mutter, feststehend gelagert ist. Die Druckfeder kann eine Zugkraft auf den Rand der Faszie bewirken, die über das Zugkraftübertragungselement, das Anbindungselement und die Zugmittel übertragen wird. Gleichzeitig kann über die Länge der gestauchten Druckfeder die über das Zugkraftübertragungselement übertragene Zugkraft ermittelt werden. Beispielsweise kann das erste Federende an dem Halteelement anliegen und/oder mit dem Halteelement verbunden sein. Das zweite Federende kann an dem Mitnehmer des Zugkraftübertragungselements oder an dem auf dem Außengewinde des Zugkraftübertragungselements aufsitzenden Befestigungselement anliegen oder mit dem Mitnehmer oder dem Befestigungselement verbunden sein. Als vorteilhaft hat es erwiesen, wenn die Druckfeder des Zugkraftmessers konzentrisch zu dem Zugkraftübertragungselement angeordnet ist. Insofern kann das Zugkraftübertragungselement sich innerhalb der Druckfeder gegenüber dem ersten Federende bewegen, welches in Bezug auf das Halteelement feststehend gelagert ist.

Gemäß einer vorteilhaften Ausgestaltung weist der Zugkraftmesser eine Skalenhülse und eine Zeigerhülse auf, die konzentrisch zu dem Zugkraftübertragungselement gegeneinander bewegbar angeordnet sind. Die Skalenhülse kann mehrere, insbesondere drei, Bereiche aufweisen, die mit unterschiedlichen Farben und/oder Schraffuren und/oder Symbolen versehen sind. Alternativ oder zusätzlich kann die Skalenhülse eine Skalenanzeige aufweisen. Bevorzugt weist die Zeigerhülse einen größeren Durchmesser auf als die Skalenhülse, so dass die Zeigerhülse die Skalenhülse von außen umgreifen kann. Bevorzugt ist die Skalenhülse an dem Haltelement und die Zeigerhülse an dem Zugkraftübertragungselement, insbesondere an einem Mitnehmer oder an einem auf einem Außengewinde des Zugkraftübertragungselements aufsitzenden Befestigungselement, befestigt. Alternativ kann die Zeigerhülse an dem Haltelement und die Skalenhülse an dem Zugkraftübertragungselement, insbesondere an einem Mitnehmer oder an einem auf einem Außengewinde des Zugkraftübertragungselements aufsitzenden Befestigungselement, befestigt sein.

Besonders bevorzugt ist es, wenn der Zugkraftmesser drei oder mehr Hülsen aufweist, welche konzentrisch zu dem Zugkraftübertragungselement gegeneinander bewegbar angeordnet sind.

Vorteilhaft ist eine Ausgestaltung, bei welcher das Zugkraftübertragungselement starr ausgebildet ist. Unter einem starren Zugkraftübertragungselement wird insbesondere ein Zugkraftübertragungselement verstanden, über welches eine der Zugkraft entgegengesetzte Druckkraft übertragbar ist. Das starre Zugkraftübertragungselement kann beispielsweise als Stab, als Schraube oder als Rohr ausgebildet sein. Alternativ kann das Zugkraftübertragungselement elastisch ausgebildet sein, beispielsweise als Schlauch.

Als vorteilhaft hat sich eine Ausgestaltung erwiesen, bei welcher das Anbindungselement mit dem Halteelement lösbar verbunden ist, so dass das Halteelement von dem Patienten entfernt werden kann, ohne dass es erforderlich wäre, die Zugmittel von dem Rand der Faszie zu lösen. Ein solches Lösen kann insbesondere dann erforderlich sein, wenn der Bereich um den Weichteildefekt freigegeben werden muss, beispielsweise um Reanimationsmaßnahmen an dem Patienten durchführen zu können.

Bevorzugt weist die Vorrichtung eine Entriegelungseinrichtung auf, über welche die Verbindung zwischen den Halteelement und dem Anbindungselement und/oder die Verbindung zwischen dem Zugkraftübertragungselement und dem Halteelement und/oder die Verbindung zwischen dem Anbindungselement und dem Zugkraftübertragungselement lösbar ist. Die Entriegelungseinrichtung ist bevorzugt als Schnellentriegelungseinrichtung ausgebildet, welche mit einem Handgriff entriegelt werden kann. Beispielsweise kann das Zugkraftübertragungselement derart mit dem Anbindungselement verbunden sein, dass es zusammen mit dem Anbindungselement von dem Halteelement lösbar ist.

Besonders bevorzugt ist es, wenn das Zugkraftübertragungselement einen ersten Teil und einen zweiten Teil aufweist, welche wahlweise miteinander verbindbar oder voneinander trennbar sind. Eine derartige Ausgestaltung hat den Vorteil, dass der erste Teil in einem sterilen Bereich angeordnet werden kann und der zweite Teil in einem nicht sterilen Bereich, oder umgekehrt.

Bevorzugt ist zwischen dem ersten Teil und dem zweiten Teil eine lösbare Kupplung angeordnet, die den ersten Teil mit dem zweiten Teil verbindet. Die Kupplung ist besonders bevorzugt per Hand lösbar, d.h. ohne ein Werkzeug, so dass der Bereich des Weichteildefekts schnell freigegeben werden kann, beispielsweise wenn Reanimationsmaßnahmen an dem Patienten durchgeführt werden sollen. Beispielsweise kann die Kupplung eine Steckkupplung oder eine Drehkupplung sein.

Bevorzugt ist das Zugkraftübertragungselement mit dem Halteelement und/oder mit dem Anbindungselement über ein Gelenk verbunden. Durch das Gelenk ist es möglich, die Lage des Anbindungselements gegenüber dem Halteelement einzustellen. Das Gelenk kann als Kugelgelenk ausgebildet sein. Alternativ kann das Gelenk als Scharniergelenk ausgestaltet sein. Bei einer Ausgestaltung, bei der das Zugkraftübertragungselement einen ersten Teil und einen zweiten Teil aufweist, zwischen denen eine Kupplung angeordnet ist, kann das Gelenk, insbesondere das Kugelgelenk, im Bereich der Kupplung vorgesehen sein. Das Gelenk kann entweder an dem ersten Teil oder an dem zweiten Teil des Zugkraftübertragungselements angeordnet sein.

Eine vorteilhafte Ausgestaltung sieht vor, dass das Anbindungselement zwei Anbindungsbereiche aufweist, wobei die Anbindungsbereiche jeweils mehrere Anbindungspunkte aufweisen und ein Abstand des ersten Anbindungsbereichs zu dem zweiten Anbindungsbereich einstellbar ist. Bevorzugt sind die beiden Anbindungsbereiche voneinander lösbar. Besonders bevorzugt sind die beiden Anbindungsbereiche voneinander und von dem Zugkraftübertragungselement lösbar.

Eine vorteilhafte Ausgestaltung sieht vor, dass das Anbindungselement zwei Anbindungsbereiche aufweist, wobei die Anbindungsbereiche jeweils mehrere Anbindungspunkte aufweisen und die Anbindungsbereiche gegeneinander bewegbar sind. Bevorzugt ist der Abstand des ersten Anbindungsbereichs zu dem zweiten Anbindungsbereich einstellbar. Die Anbindungsbereiche können über eine oder mehrere teleskopierbare Verbindungselemente, insbesondere teleskopierbare Stangen, verbunden sein. Bevorzugt ist die Stellung der Anbindungsbereiche zueinander arretierbar. Das Zugkraftübertragungselement kann mit einem der Anbindungsbereiche verbunden sein. Bevorzugt ist ein erster Anbindungsbereich mit dem Zugkraftübertragungselement derart verbunden, dass ein zweiter Anbindungsbereich gegenüber dem ersten Anbindungsbereich und dem Zugkraftübertragungselement bewegbar ist. Die Anbindungsbereiche können als Stangen oder Rohre ausgebildet sein. Die Anbindungsbereiche können gerade ausgebildet sein oder eine Krümmung aufweisen. Beispielsweise ist es möglich, dass zwei gerade Anbindungsbereiche vorgesehen sind welche parallel zu einander verlaufend angeordnet sind. Alternativ ist es möglich, dass zwei gekrümmte Anbindungsbereiche vorgesehen sind.

In diesem Zusammenhang ist es vorteilhaft, wenn das Anbindungselement ein Basisteil aufweist und die Anbindungsbereiche gegenüber dem Basisteil bewegbar sind. Bevorzugt ist das Basisteil mit dem Zugkraftübertragungselement verbunden. Bevorzugt ist das Basisteil mit dem Zugkraftübertragungselement über ein Gelenk, insbesondere ein Kugelgelenk oder ein Scharniergelenk, verbunden. Bevorzugt ist die Stellung der Anbindungsbereiche gegenüber dem Basisteil arretierbar. Die Anbindungsbereiche können mit dem Basisteil über teleskopierbare Verbindungselemente, insbesondere teleskopierbare Stangen, verbunden sein.

Eine bevorzugte Ausgestaltung sieht vor, dass die Anbindungsbereiche von dem Basisteil lösbar sind. Bei einer derartigen Ausgestaltung können die Anbindungsbereiche von dem Basisteil getrennt werden, um einen verbesserten Zugang zu dem offenen Weichteildefekt zu erhalten. Es ist daher möglich, an dem Weichteildefekt zu arbeiten, ohne die Zugmittel von den Anbindungsbereichen lösen zu müssen.

Bevorzugt ist eine Ausgestaltung, bei der das Anbindungselement ein Basisteil aufweist, wobei die Anbindungsbereiche von dem Basisteil lösbar sind und an dem Basisteil mehrere Verbindungsstellen vorgesehen sind, mit denen die Anbindungsbereiche wahlweise verbindbar sind, so dass verschiedene Abstände zwischen den Anbindungsbereichen eingestellt werden können.

Besonders bevorzugt weist das Basisteil an den Verbindungsstellen Durchgangsbohrungen auf, in denen jeweils ein Sicherungselement, insbesondere ein Sicherungsbolzen oder eine Sicherungsschraube, angeordnet ist. Der Sicherungsbolzen kann zwischen einer Entriegelungsstellung, in welcher der Anbindungsbereich von dem Basisteil entfernbar ist, und einer Verriegelungsstellung bewegbar sein, in welcher der Anbindungsbereich über den Sicherungsbolzen an dem Basisteil festgelegt ist. Der Sicherungsbolzen kann über ein Federelement vorgespannt sein, insbesondere in Richtung der Verriegelungsstellung. Besonders bevorzugt ist der Sicherungsbolzen als über ein Federelement vorgespannter Rastbolzen ausgebildet, der in der Entriegelungsstellung wahlweise eingerastet werden kann.

Vorteilhaft ist es, wenn die Vorrichtung zumindest ein mit der Faszie verbindbares Zugmittel aufweist. Über das Zugmittel kann die Zugkraft in die Faszie eingeleitet werden. Bevorzugt sind mehrere Zugmittel vorgesehen, über welche Zugkräfte an mehrere verschiedenen Stellen auf die Ränder der geöffneten Faszie aufgebracht werden können. Die verschiedenen Zugmittel können jeweils unterschiedlich starke und unterschiedlich ausgerichtete Zugkräfte in die Faszie einleiten. Alternativ kann das Zugmittel mit der Körperoberfläche, insbesondere mit der Bauchdecke oder Rückenwand, einschließlich der Faszie, verbunden sein, so dass nicht nur die Faszie, sondern auch die über der Faszie liegende Haut auf Spannung gehalten wird. Bevorzugt ist das Zugmittel als Faden und/oder als Draht und/oder als Netz ausgebildet. Es können herkömmliche, in der Chirurgie verwendete Fäden, Drähte oder Netze zur Anwendung kommen. Das Zugmittel kann direkt oder indirekt mittels eines Verbindungsmittels an der Faszie angebunden sein. Bevorzugt ist das Zugmittel als eine Kombination eines Fadens und/oder eines Drahts und/oder eines Netzes ausgebildet. Bevorzugt ist es ferner, wenn das Zugmittel über ein flächig ausgebildetes Verbindungsmittel mit der Faszie verbindbar ist. Über das flächige Verbindungsmittel kann eine verbesserte Anbindung des Zugmittels an die Faszie erreicht werden. Die Gefahr des Ausreißens der Faszie kann vermindert werden und die Faszie kann beim wiederholten Wechseln des Zugmittels geschont werden. Besonders bevorzugt ist eine Ausgestaltung, bei welcher das flächige Verbindungsmittel mit der Faszie vernähbar ist.

Alternativ kann das Verbindungsmittel beispielsweise Haken, insbesondere Widerhaken, aufweisen, welche in die Faszie eingebracht werden können. Das Verbindungsmittel kann beispielsweise als Netz oder als Platte ausgebildet sein. Das Zugmittel kann über das flächig ausgebildete Verbindungsmittel mit der Faszie und zusätzlich mit anderen Bestandteilen des Weichteilmantels, bevorzugt mit dem gesamten Weichteilmantel, verbindbar sein. Beispielsweise kann neben der Faszie auch die Haut mit dem flächigen Verbindungsmittel verbunden sein. Vorteilhaft ist es, wenn das flächig ausgebildete Verbindungsmittel reversibel mit dem Zugmittel verbindbar ist, so dass das Zugmittel und/oder die Aufhängung problemlos entfernt und wieder angebracht werden kann, um den offenen Weichteildefekt, insbesondere das offene Abdomen oder den offenen Rücken, zu behandeln.

Eine vorteilhafte Ausgestaltung sieht vor, dass das Anbindungselement im Bereich der Anbindungspunkte jeweils eine Durchgangsbohrung und eine in die Durchgangsbohrung mündende Einführnut aufweist, über welche ein Zugmittel in die Durchgangsbohrung einführbar ist. Dies bringt den Vorteil mit sich, dass ein in der Durchgangsbohrung ggf. angeordnetes Sicherungselement, insbesondere ein Bolzen oder eine Schraube, nicht vollständig aus der Durchgangsbohrung entfernt werden muss, um das Zugmittel in die Durchgangsbohrung einzubringen. Die Anbindungspunkte sind bevorzugt in den Anbindungsbereichen des Anbindungselements angeordnet.

Eine alternative Ausgestaltung der Anbindungspunkte sieht vor, dass diese eine konische Durchgangsöffnung aufweisen, in der eine Kugel angeordnet ist, über welche ein durch die Durchgangsöffnung geführtes Zugmittel in der Durchgangsöffnung einklemmbar ist. Die Kugel kann über ein Sicherungselement gegen ein unerwünschtes Herausfallen aus der konischen Durchgangsbohrung gesichert sein. Optional kann eine Einführnut vorgesehen sein, die in der konischen Durchgangsbohrung mündet.

Zur Lösung der eingangs genannten Aufgabe trägt ferner ein Kit zur Behandlung eines offenen Weichteildefekts mit mindestens einem Schwamm zum Ausfüllen des Weichteildefekts bei, wobei das Kit eine vorstehend beschriebene Vorrichtung aufweist.

Hierdurch können dieselben Vorteile erreicht werden, wie sie bereits im Zusammenhang mit der erfindungsgemäßen Vorrichtung zur Verminderung der Retraktion der Ränder einer geöffneten Faszie eines Patienten beschrieben wurden.

Eine vorteilhafte Ausgestaltung des Kits sieht vor, dass das Kit zusätzlich ein Abdichtmittel zum Abdichten des offenen Weichteildefekts, insbesondere des Abdomens oder des offenen Rückens, aufweist. Das Abdichtmittel kann als Folie ausgebildet sein, welche auf der dem Körper abgewandten Seite des Schwamms auf den offenen Weichteildefekt, insbesondere die geöffnete Bauchdecke oder die geöffnete Rückenwand, aufgebracht, insbesondere aufgeklebt, werden kann. Durch das Abdichtmittel kann der Körperinnenraum, insbesondere der Bauchraum oder der innerhalb der Rückenwand liegende Raum, flüssigkeitsdicht und gasdicht verschlossen werden. Das Abdichtmittel kann derart angeordnet werden, dass die Vorrichtung zur Verminderung der Retraktion der Ränder einer geöffneten Faszie ganz oder teilweise innerhalb des durch das Abdichtmittel abgedichteten Bereichs liegt. Bevorzugt ist das Abdichtmittel als nicht-klebende, luftundurchlässige Folie ausgebildet. Die nicht-klebende, luftundurchlässige Folie kann unmittelbar an der Vorrichtung zur Verminderung der Retraktion der Faszienränder anliegen. Die nicht-klebende, luftundurchlässige Folie kann über Klebemittel, beispielsweise eine Klebefolie, an dem Körper des Patienten fixiert werden.

Das Abdichtmittel weist vorteilhafterweise eine Öffnung zum Durchführen des Zugkraftübertragungselements auf. Im Bereich der Öffnung des Abdichtmittels kann das Abdichtmittel mit dem Dichtelement des Zugkraftübertragungselements verbindbar sein. Das Dichtelement kann als, insbesondere elastischer, Dichtring ausgebildet sein. Bevorzugt weist das Abdichtmittel, insbesondere die Folie, im Bereich der Öffnung ein Klebemittel auf, über welches das Abdichtmittel dichtend mit dem Dichtelement des Zugkraftübertragungselements verbunden werden kann. Das Klebemittel kann beispielsweise als selbstklebender Bereich des Abdichtmittels oder als an dem Abdichtmittel angeordnetes klebendes Element ausgebildet sein.

Bevorzugt weist das Kit eine Vakuumpumpe zur Erzeugung eines Unterdrucks im Körperinnenraum, insbesondere im Bauchraum oder im innerhalb der Rückenwand liegenden Raums, des Patienten auf. Über die Vakuumpumpe können überschüssige Flüssigkeiten und/oder Gase aus dem Körperinnenraum, insbesondere dem Bauchraum oder dem innerhalb der Rückenwand liegenden Raum, abgesaugt werden.

### Kurze Beschreibung der Zeichnungen

Weitere Vorteile und Einzelheiten der Erfindung sollen nachfolgend anhand der in den Figuren dargestellten Ausführungsbeispiele beschrieben werden. Darin zeigt:
- Fig. 1: eine schematische Schnittdarstellung eines menschlichen Abdomens entlang einer Sagittalebene mit einer Vorrichtung gemäß einem Ausführungsbeispiel der Erfindung bei geöffnetem Abdomen;
- Fig. 2: eine schematische Seitenansicht der Vorrichtung gemäß Fig. 1;
- Fig. 3: eine schematische Schnittdarstellung eines menschlichen Abdomens mit einem erfindungsgemäßen Kit zur Behandlung eines offenen Abdomens mit einer der Vorrichtung nach Fig. 1;
- Fig. 4: eine schematische Schnittdarstellung eines menschlichen Abdomens mit einem erfindungsgemäßen Kit zur Behandlung eines offenen Abdomens mit einer Vorrichtung gemäß einem zweiten Ausführungsbeispiel;
- Fig. 5: eine schematische Schnittdarstellung eines menschlichen Abdomens mit einem erfindungsgemäßen Kit zur Behandlung eines offenen Abdomens mit einer Vorrichtung gemäß einem dritten Ausführungsbeispiel;
- Fig. 6: eine schematische Schnittdarstellung eines Anbindungsbereichs einer Vorrichtung gemäß einem Ausführungsbeispiel der Erfindung;
- Fig. 7: eine schematische Schnittdarstellung eines Anbindungspunkts in einem Anbindungsbereich nach Fig. 6;
- Fig. 8: eine Aufsicht auf ein Anbindungselement einer Vorrichtung gemäß einem Ausführungsbeispiel der Erfindung in einer ersten Konfiguration;
- Fig. 9: eine Aufsicht auf das Anbindungselement nach Fig. 8 in einer zweiten Konfiguration;
- Fig. 10: eine schematische Schnittdarstellung einer Vorrichtung gemäß einem vierten Ausführungsbeispiel der Erfindung; und
- Fig. 11: eine schematische Schnittdarstellung einer Vorrichtung gemäß einem fünften Ausführungsbeispiel der Erfindung.

### Ausführungsbeispiele der Erfindung

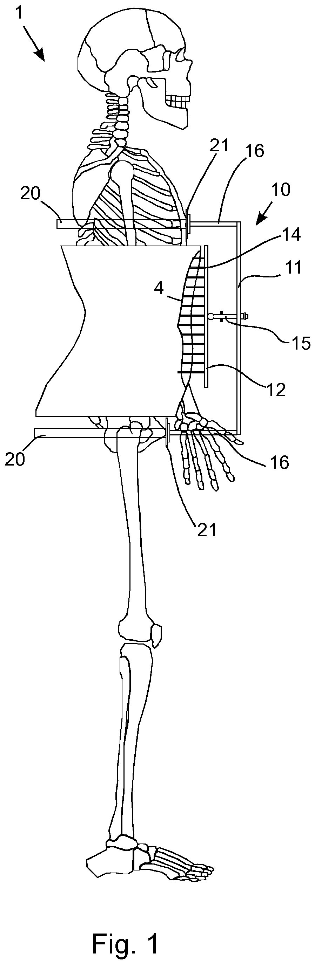

In der **Fig. 1** ist eine schematische Schnittdarstellung eines Patienten 1 entlang einer Sagittalebene dargestellt. Der Patient 1 weist einen Weichteildefekt in Form eines geöffneten Abdomens auf. Bei einigen Krankheitsbildern, welche mit einer intraabdominellen Druckerhöhung, einem abdominellen Kompartmentsyndrom, einhergehen, ist es erforderlich, die Bauchdecke des Patienten 1 durch chirurgische Maßnahmen zu öffnen. Eine solche Therapie mit offenem Abdomen ermöglicht, den Druck innerhalb des Bauchraums zu senken und überschüssige Gase und/oder Flüssigkeiten aus dem Bauchraum zu entfernen. Hierbei kann es zu einem Heraustreten innenliegender Organe, beispielsweise des Darms, durch die Bauchdeckenöffnung kommen. Um der Entstehung von Infektionen im
Bauchraum entgegenzuwirken und die Pflege des offenen Abdomens zu erleichtern, wird die Bauchdeckenöffnung in der Regel provisorisch abgedichtet und gleichzeitig eine Unterdrucktherapie durchgeführt, auf welche nachfolgend noch näher eingegangen wird. Eine solche Unterdrucktherapie wird in der Regel über eine Dauer von einigen Tagen bis hin zu mehreren Wochen durchgeführt. Damit sich die Ränder der Faszie 4 in dieser Zeitspanne nicht übermäßig zurückziehen und das Schließen der Faszie 4, d.h. das Verbinden der Faszienränder, nach der Periode des offenen Abdomens mit unproblematischer Wundheilung möglich ist, wird nach dem Öffnen der Bauchdecke eine erfindungsgemäße Vorrichtung 10 zur Verminderung der Retraktion der Faszie 4 eingesetzt.

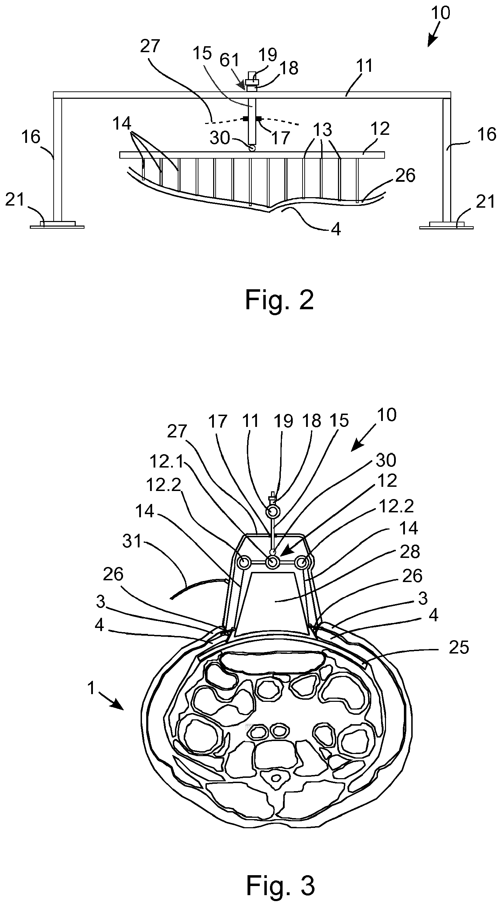

Wie den Darstellungen in **Fig. 1** **und** **2** entnommen werden kann, weist die Vorrichtung 10 zur Verminderung der Retraktion der Faszie 4 ein Halteelement 11 auf, welches über mehrere, insbesondere zwei, als Stützen 16 ausgebildete Befestigungseinrichtungen an dem Patienten festlegbar ist. Insofern kann das Haltelement 11 in einem ersten Abstand von dem Patienten 1 festgelegt werden. Optional können die Stützen 16 über eine Gurtbefestigung 20 am Körper des Patienten befestigt werden, so dass ein ungewolltes Verrutschen der Stützen 16 verhindert wird. Das Halteelement 11 ist als Stange ausgebildet. In dem Ausführungsbeispiel ist das Halteelement 11 fest mit den beiden Stützen 16 verbunden. Alternativ kann zur Verbindung des Halteelements 11 mit einer Stütze 16 jeweils ein Gelenk zwischen dem Halteelement 11 und der jeweiligen Stütze 16 angeordnet sein. Die Stützen 16 können optional längenveränderlich ausgestaltet sein, beispielsweise teleskopierbar. An ihrer dem Halteelement 11 abgewandten Seite weisen die Stützen 16 jeweils ein als Halteplatte ausgebildetes Druckverteilungsmittel 21 auf, welches auf der Hautoberfläche des Patienten 1 aufliegt. Über das Druckverteilungsmittel 21 wird eine vergrößerte Auflagefläche bereitgestellt, so dass die Gefahr der Bildung von Druckstellen auf der Haut des Patienten 1 reduziert wird.

Die Vorrichtung 10 weist ferner ein mit dem Halteelement 11 verbundenes Anbindungselement 12 auf. Das Anbindungselement 12 ist in einem zweiten Abstand zu dem Patienten 1 angeordnet, welcher kleiner ist als der Abstand des Halteelements 11 von dem Patienten 1. Das bedeutet, dass das Anbindungselement 12 näher an dem Patienten 1 angeordnet ist als das Halteelement 11. An dem Anbindungselement 12 sind mehrere Anbindungspunkte 13 vorgesehen, welche derart ausgebildet sind, dass an ihnen Zugmittel 14 angebunden werden können, die mit dem Rand der geöffneten Faszie 4 verbunden sind. Über die Zugmittel 14 werden Zugkräfte auf die Ränder der Faszie 4 übertragen. Die Zugmittel 14 sind vorzugsweise als Fäden ausgebildet. Alternativ können Drähte, Seile oder Netze verwendet werden. Die Zugmittel 14 können elastisch oder nicht-elastisch ausgebildet sein. Grundsätzlich ist es möglich, die Zugmittel 14 unmittelbar mit der Faszie 4 zu verbinden. Um ein Ausreißen der Zugmittel 14 und eine Schädigung der Faszie 4 zu verhindern, werden die Zugmittel 14 vorzugsweise über ein als Netz 26 ausgebildetes, flächiges Verbindungsmittel mit der Faszie 4 verbunden. Das flächige Verbindungsmittel kann die erheblichen Zugkräfte auf eine größere Fläche verteilen und damit die Einleitung der Zugkraft in die Faszie 4 verbessern. Das flächige Verbindungsmittel kann über die gesamte Dauer der Behandlung des offenen Abdomens mit der Faszie 4 verbunden bleiben. Die Zugmittel 14 können hingegen bei Bedarf ausgetauscht werden.

Über die Vorrichtung 10 werden Zugkräfte mit einer vom Körper des Patienten 1 weg gerichteten Kraftkomponente derart auf die Ränder der geöffneten Faszie 4 aufgebracht, dass die Vorrichtung 10 die Ränder auf Spannung und gleichzeitig voneinander beabstandet hält. Durch die Zugkraft wird die Spannung der Faszie 4 während der Therapie mit offenem Abdomen aufrechterhalten und damit die Retraktion der Ränder der Faszie 4 verhindert. Der Weichteildefekt und damit auch die Faszie 4, wird offen gehalten. Der Weichteildefekt hat eine Ausdehnung von mehr als 1 cm, bevorzugt von mehr als 5 cm, so dass ein ungehinderter Zugang zum Körperinneren möglich ist. Die Ränder der Faszie 4 können nach Abschluss der Therapie aneinander angelegt und direkt verschlossen werden.

Erfindungsgemäß ist vorgesehen, dass das Anbindungselement 12 mit dem Halteelement 11 über genau ein Zugkraftübertragungselement 15 verbunden ist, so dass die Abdichtung des Unterdruckbereichs bei der Unterdrucktherapie erleichtert wird. Das Zugkraftübertragungselement 15 ist als starres Zugkraftübertragungselement 15, beispielsweise als massive Stange oder hohles Rohr, ausgebildet. Das Zugkraftübertragungselement 15 ist mit dem Anbindungselement 12 über ein Gelenk 30, insbesondere ein Kugelgelenk, verbunden, so dass die Ausrichtung des Anbindungselements 12 gegenüber dem Zugkraftübertragungselement 15 verändert werden kann. Eine den Unterdruckbereich abgrenzende Folie 27 muss nur an einer Stelle gegenüber dem Zugkraftübertragungselement 15 abgedichtet werden. An dem Zugkraftübertragungselement 15 ist ferner ein Dichtelement 17 angeordnet, an welchem die Folie 27 befestigt werden kann. Die Folie 27 weist bevorzugt eine Öffnung auf, durch welche das Zugkraftübertragungselement 15 geführt werden kann. Der Rand der Öffnung ist bevorzugt als Kleberand ausgebildet. Die Folie 27 kann mit dem Dichtelement 17 über den Kleberand dichtend verbunden werden. Das Dichtelement 17 kann beispielsweise als Dichtring ausgestaltet sein. Das Dichtelement 17 ist gegenüber dem Zugkraftübertragungselement 15 drehbar und/oder entlang des Zugkraftübertragungselements 15 verschiebbar. Insofern ist es möglich, die Lage des Zugkraftübertragungselements 15 zu verändern, ohne die Folie 27 zu beeinträchtigen. Damit wird es möglich, während der Unterdrucktherapie ohne großen Aufwand Veränderungen an der Ausrichtung des Anbindungselements 12 und/oder des Zugkraftübertragungselements 15 vorzunehmen.

Das Zugkraftübertragungselement 15 ist gegenüber dem Halteelement 11 derart bewegbar, das über die Bewegung des Zugkraftübertragungselements 15 der Abstand zwischen dem Anbindungselement 12 und dem Halteelement 11 eingestellt werden kann. Das Zugkraftübertragungselement 15 ist in einer als Durchgangsöffnung ausgebildeten Ausnehmung 61 in dem Halteelement 11 gelagert. Das Zugkraftübertragungselement 15 weist in einem oberen Bereich, welcher dem Anbindungselement 11 gegenüber liegt, ein Außengewinde auf. Auf dem Außengewinde sitzt ein Befestigungselement 19 auf. Das Befestigungselement 19 weist ein Innengewinde auf. Beispielsweise kann das Befestigungselement 19 als Mutter ausgebildet sein. Zwischen dem Befestigungselement 19 und dem Halteelement 11 ist eine Druckfeder angeordnet, die in Abhängigkeit von der über das Zugkraftübertragungselement 15 übertragenden Zugkraft zusammengedrückt wird. Die Druckfeder kann Teil eines Zugkraftmessers 18, insbesondere einer Federwaage, sein, welche die über das Zugkraftübertragungselement 15 übertragene Zugkraft anzeigt.

Das Befestigungselement 19 bildet ein Betätigungselement, über welches die gesamte auf die Ränder der Faszie 4 wirkende Zugkraft eingestellt werden kann. Das Befestigungselement 19 ist im oberen Bereich des Zugkraftübertragungselements 15 angeordnet, welcher dem Anbindungselement 12 gegenüberliegt, so dass das Befestigungselement 19 auch dann betätigt werden kann, wenn eine Unterdrucktherapie durchgeführt wird. Bevorzugt ist das Befestigungselement 19 derart ausgebildet, dass es von einer Eingriffsstellung, in welcher das Innengewinde des Befestigungselements 19 in Eingriff mit dem Außengewinde des Zugkraftübertragungselements 15 steht, in eine Offenstellung verbringbar ist, in welcher das Innengewinde des Befestigungselements 19 nicht in Eingriff mit dem Außengewinde des Zugkraftübertragungselements 15 steht. Beispielsweise kann das Befestigungselement 19 als geteilte Mutter, insbesondere aufschwenkbare Mutter ausgebildet sein.

An dem Befestigungselement 19 ist eine Entriegelungseinrichtung vorgesehen, über welche das Befestigungselement 19 mit einem Handgriff in die Offenstellung verbracht werden kann. In der Offenstellung kann das Befestigungselement 19 von dem Zugkraftübertragungselement 15 entfernt werden. In einem weiteren Schritt kann das Halteelement 11 von dem Patienten 1 entfernt werden, ohne dass es erforderlich wäre, die Zugmittel 14 von dem Rand der Faszie 4 zu lösen. Das über die Zugmittel 14 mit dem Patienten1 verbundene Anbindungselement 12 sowie das mit dem Anbindungselement verbundene Zugkraftübertragungselement 15 verbleibt am Patienten. Ein solches Lösen kann insbesondere dann erforderlich sein, wenn der Bereich des Weichteildefekts freigegeben werden muss, beispielsweise um den Patienten zu reanimieren.

Wie die Darstellung in **Fig. 3** entnommen werden kann, weist das Anbindungselement 12 ein Basisteil 12.1 auf, welches mit dem Zugkraftübertragungselement 15 verbunden ist. Mit dem Basisteil 12.1 sind zwei Anbindungsbereiche 12.2 derart verbunden, dass diese gegenüber dem Basisteil 12.1 und gegeneinander bewegbar sind. Die Anbindungsbereiche 12.2 können gegenüber dem Basisteil 12.1 arretiert werden. An den Anbindungsbereichen 12.2 sind jeweils mehrere Anbindungspunkte 13 für Zugmittel 14 vorgesehen. Die Anbindung der Zugmittel 14 an die Anbindungspunkte 13 kann dadurch erfolgen, dass die Zugmittel 14 durch eine in den Figuren nicht dargestellte Durchgangsöffnung in dem Anbindungsbereich geführt und dann beispielsweise verknotet werden. Eine alternative Anbindung der Zugmittel 14 kann über als Rillen ausgebildete Anbindungspunkte 13 erfolgen. Die Zugmittel 14 können vorteilhafterweise durch die Rillen geführt werden.

Das Basisteil 12.1 und die Anbindungsbereiche 12.2 sind als Stangen ausgebildet, welche insbesondere parallel zueinander angeordnet sind. Zur Verbindung der Anbindungsbereiche 12.2 mit dem Basisteil 12.1 sind bei dem Ausführungsbeispiel teleskopierbare Querstangen zwischen dem Basisteil 12.1 und den Anbindungsbereichen 12.2. angeordnet.

Bei Durchführung der Unterdrucktherapie wird der Bauchraum beispielsweise über einen unterhalb der Bauchdecke angeordneten Schwamm 25 abgedeckt. Zwischen dem Schwamm 25 und dem Darm wird bevorzugt eine in den Figuren nicht dargestellte Folie oder ein Trennmaterial angeordnet, um ein Austrockenen des Darms zu verhindern. In die Bauchdeckenöffnung wird ein vorzugsweise weiterer Schwamm 28 eingebracht und die Bauchdeckenöffnung wird mit einem Abdichtmittel 30, welches üblicherweise als gasdichte Folie 27 ausgebildet ist, abgedichtet. Der Schwamm 28 wird über einen Saugschlauch 31 mit einer Vakuumpumpe verbunden. Über die Vakuumpumpe wird ein Unterdruck angelegt, so dass durch den Saugschlauch 31 unerwünschte Flüssigkeiten und/oder Gase aus dem Bauchraum entfernt werden können. Diese Unterdrucktherapie wird in der Regel über eine Dauer von einigen Tagen bis hin zu mehreren Wochen durchgeführt.

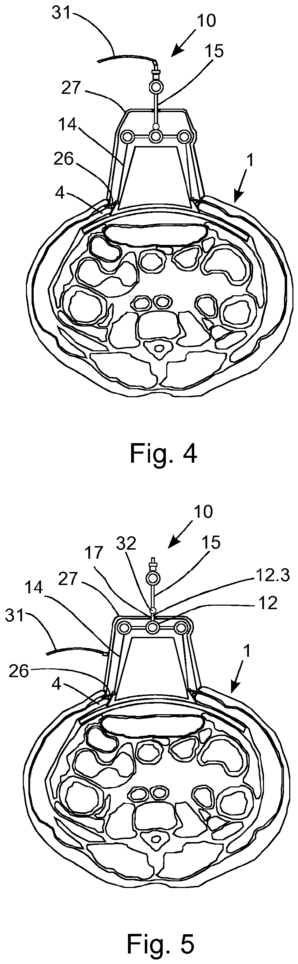

Gemäß einer in **Fig. 4** gezeigten alternativen Ausführungsbeispiels weist das Zugkraftübertragungselement 15 einen Luftkanal auf. Der Luftkanal kann eine erste Öffnung zum Anschluss einer Vakuumpumpe mit einer im Bereich des Anbindungselements 12 angeordneten, zweiten Öffnung verbinden. Bei einer derartigen Ausgestaltung ist es möglich den Saugschlauch 31 der Vakuumpumpe mit der ersten Öffnung des Zugkraftübertragungselements 15 zu verbinden und Luft bzw. Flüssigkeit durch den Luftkanal in dem Zugkraftübertragungselement 15 aus dem Unterdruckbereich abzusaugen.

Die **Fig. 5** zeigt ein drittes Ausführungsbeispiel einer Vorrichtung 10, wobei das Anbindungselement 12 mit dem Zugkraftübertragungselement 15 über ein Gelenk 32, beispielsweise ein Kugelgelenk oder Scharniergelenk, verbunden ist. Im Unterschied zu den vorher beschriebenen Ausführungsbeispielen ist an dem Anbindungselement 12 ein Dichtelement 17, z. B. ein Dichtring, vorgesehen. Das Anbindungselement kann einen, insbesondere stangenartigen, Dichtbereich 12.3 aufweisen, an welchem der Dichtring 17 angeordnet ist.

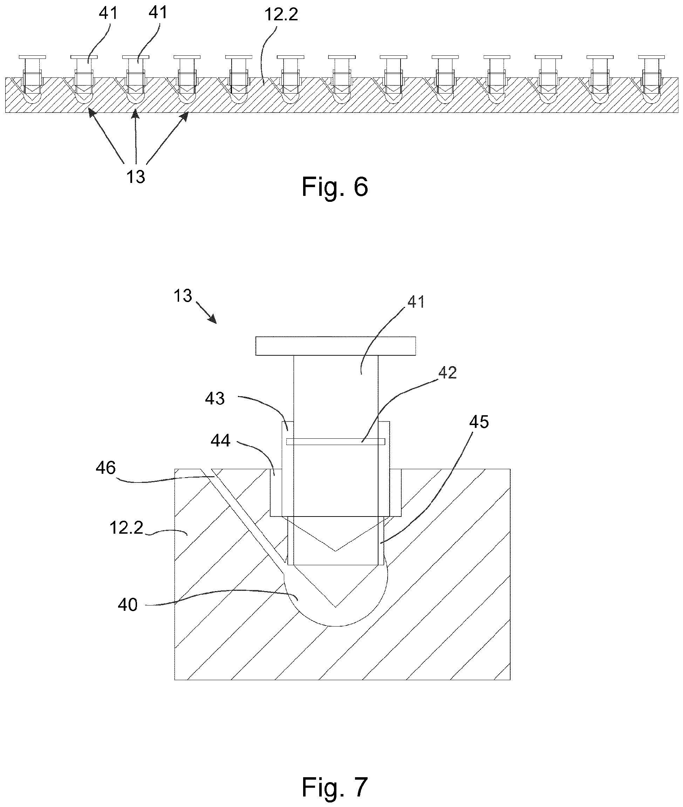

In der **Fig. 6** ist ein Ausführungsbeispiel eines Anbindungsbereichs 12.2 eines Anbindungselementes 12 dargestellt, wie er bei der erfindungsgemäßen Vorrichtung 10 Verwendung finden kann. Der Anbindungsbereich 12 weist mehrere, hier dreizehn, Anbindungspunkte 13 auf, an denen jeweils ein Zugmittel 14 angebunden werden kann. Die Anbindungspunkte 13 sind in identischem Abstand zu einander angeordnet und weisen jeweils ein Sicherungselement 41 auf, über welches das Zugmittel 14 an dem Anbindungspunkt 13 festgelegt werden kann.

Die **Fig. 7** zeigt eine vergrößerte Darstellung eines Anbindungspunkts 13 in dem Anbindungsbereich 12.2 nach Fig. 6. In dem Anbindungsbereich 12.2 des Anbindungselements 12 ist eine Durchgangsbohrung 40 angeordnet. Ferner ist eine Einfuhrnut 46 vorgesehen, über welche ein Zugmittel 14, beispielsweise ein Faden, in die Durchgangsbohrung 40 eingeführt werden kann. Die Einfuhrnut 46 beginnt an einer Oberfläche des Anbindungsbereichs 12.2 und mündet in der Durchgangsbohrung 40. In der Durchgangsbohrung 40 ist ein als Schraube ausgebildetes Sicherungselement 41 angeordnet. Ein Außengewinde 45 des Sicherungselements 41 wirkt mit einem an dem Anbindungsbereich 12.2 vorgesehenen Innengewinde zusammen, so dass das Sicherungselement 41 wahlweise in die Durchgangsbohrung 40 eingedreht werden kann, um das Zugmittel 14 festzuklemmen, oder aus der Durchgangsbohrung 40 herausgedreht werden kann, um das Zugmittel 14 freizugeben.

Konzentrisch zu dem Sicherungselement 41 ist eine Führungshülse 43 angeordnet, welche über ein Gewinde 44 mit dem Anbindungsbereich 12.2 verbunden ist. An dem Sicherungselement 41 ist ferner ein Verlustschutzelement 42, beispielsweise ein Ring, angeordnet, welcher mit einer Innenkontur der Führungshülse 43 derart zusammenwirkt, dass das Sicherungselement 41 nicht von dem Anbindungsbereich 12.2 entfernt werden kann. Hierdurch wird ein unerwünschtes Herausfallen des Sicherungselements 41 verhindert.

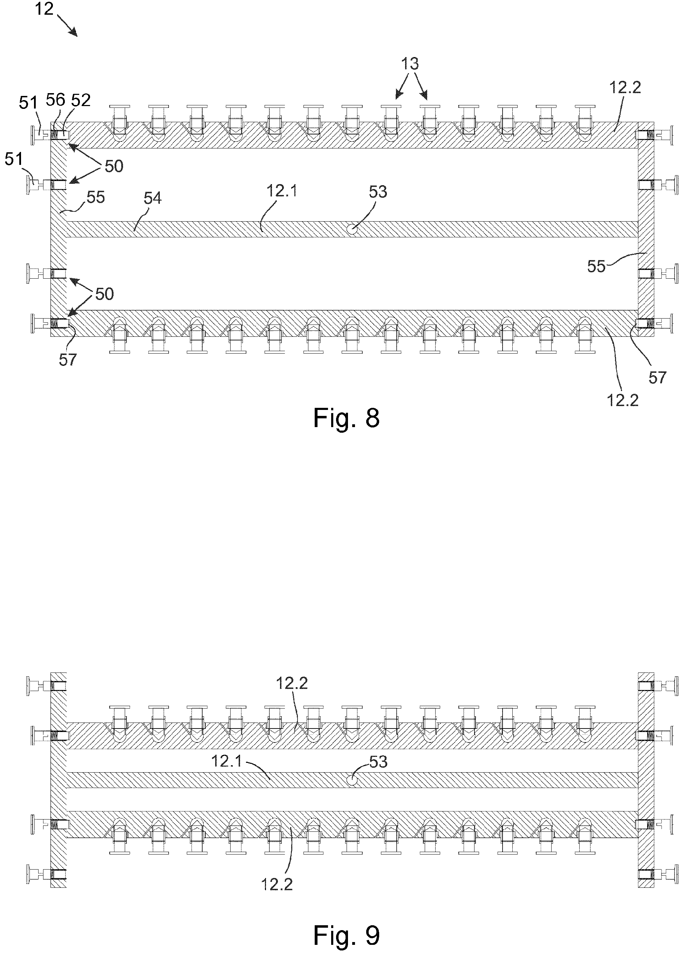

In der **Fig. 8** ist ein weiteres Ausführungsbeispiel eines Anbindungselements 12, welches bei der Vorrichtung 10 gemäß der Erfindung verwendet werden kann. Das Anbindungselement 12 weist ein Basisteil 12.1 und zwei von dem Basisteil 12.1 lösbare Anbindungsbereiche 12.2 auf. Das Basisteil 12.1 ist an einem Anbindungspunkt 53 mit dem hier nicht dargestellten einzigen Zugkraftübertragungselement 15 verbunden. Der Anbindungspunkt 53 befindet sich in einem ersten Abschnitt 54, der im Wesentlichen parallel zu den Anbindungsbereichen 12.2 verlaufend angeordnet ist. Mit dem ersten Abschnitt 54 sind zwei zweite Abschnitte 55 verbunden, die im Wesentlichen senkrecht zu den Anbindungsbereichen 12.2 verlaufend angeordnet sind.

Das Basisteil 12.1 weist, insbesondere in den zweiten Abschnitten 55 des Basisteils 12.1 mehrere Verbindungsstellen 50 auf, mit denen die Anbindungsbereiche 12.2 wahlweise verbunden werden können. Die Verbindungsstellen 50 umfassen jeweils eine Durchgangsbohrung 52, in welcher ein Sicherungsbolzen 51 aufgenommen ist. Die Sicherungsbolzen 51 können zwischen einer Entriegelungsstellung, in welcher der Anbindungsbereich 12.2 von dem Basisteil 12.1 entfernbar ist, und einer Verriegelungsstellung bewegt werden, in welcher der Anbindungsbereich 12.2 über den Sicherungsbolzen 51 an dem Basisteil 12.1 festgelegt ist. Hierzu ist an dem Anbindungsbereich 12.2 ein Sackloch 57 angeordnet, in welches ein Verriegelungsende des Sicherungsbolzens 51 in der Verriegelungsstellung eingreift. Der Sicherungsbolzen 51 ist über ein Federelement 56 in Richtung der Verriegelungsstellung vorgespannt. Ferner kann der Sicherungsbolzen 51 in der Entriegelungsstellung, insbesondere durch eine Drehbewegung, wahlweise eingerastet werden, so dass das Verriegelungsende des Sicherungsbolzens 51 nicht aus der Durchgangsbohrung 52 heraussteht.

Die Verbindungsstellen 50 sind an den beiden zweiten Abschnitten 55 des Basisteils 12.1 derart angeordnet, dass ein Anbindungsbereich 12.2 jeweils zwischen zwei gegenüberliegenden Verbindungsstellen 50 angeordnet und mit den Verbindungsstellen 50 verbunden werden kann. Es ist somit möglich, die Verbindungsbereiche 12.2 wahlweise an verschiedenen Verbindungsstellen 50 an das Basisteil 12.1 anzubinden, um den Abstand zwischen den Verbindungsbereichen 12.1 zu verändern. Hierdurch kann eine Anpassung der Vorrichtung 10 an die Größe des Weichteildefekts des Patienten 1 erfolgen. Beispielsweise zeigt die **Fig. 8** eine erste Konfiguration des Anbindungselements 12, in welcher die Anbindungsbereiche 12.2 einen größeren Abstand aufweisen, und **Fig. 9** zeigt eine zweite Konfiguration des Anbindungselements 12, in welcher die Anbindungsbereiche 12.2 einen geringeren Abstand aufweisen.

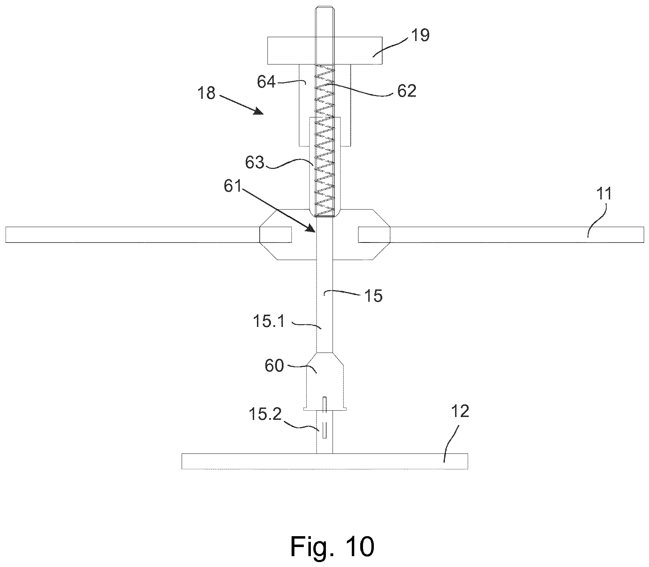

Die **Fig. 10** zeigt ein weiteres Ausführungsbeispiel einer erfindungsgemäßen Vorrichtung 10, bei welcher das Anbindungselement 12 mit dem Halteelement 11 über genau ein Zugkraftübertragungselement 15 verbunden ist. Die Vorrichtung 10 weist mehrere nicht gezeigte Stützen auf, welche das Halteelement 11 jeweils mit einem auf die Haut des Patienten auflegbaren Druckverteilungsmittel verbinden. Alternativ können die Stützen das Halteelement 11 mit einem Implantat oder mehreren Implantaten verbinden, die an einem Knochen des Patienten festgelegt sind.

Das Zugkraftübertragungselement 15 ist als starres Zugkraftübertragungselement 15 mit zwei starren Teilen 15.1, 15.2 ausgebildet. Der erste Teil 15.1 und der zweite Teil 15.2 sind wahlweise miteinander verbindbar oder voneinander trennbar. Hierzu ist zwischen dem ersten Teil 15.1 und dem zweiten Teil 15.2 eine lösbare Kupplung 60 angeordnet, die den ersten Teil 15.1 mit dem zweiten Teil 15.2 verbindet. Die die Kupplung 60 ist als eine per Hand lösbare Schnelltrennkupplung ausgebildet, beispielsweise als Steckkupplung. Im Normalfall sind die beiden Teile 15.1, 15. 2 des Zugkraftübertragungselements 15 über die Kupplung 50 miteinander verbunden. Müssen bei dem Patienten 1 jedoch Reanimationsmaßnahmen durchgeführt werden, kann die Kupplung 60 mit nur einem Handgriff gelöst werden, so dass der Zugang zu dem Patienten 1 nicht durch das Zugkraftübertragungselement 15 beeinträchtigt wird.

Das Zugkraftübertragungselement 15 ist bei der Vorrichtung gemäß Fig. 10 in einer Ausnehmung 61 in dem Halteelement 11 geführt. Das Zugkraftübertragungselement 15 weist in einem oberen Bereich, welcher dem Anbindungselement 11 gegenüber liegt, ein Außengewinde auf. Auf dem Außengewinde sitzt ein Befestigungselement 19 auf. Das Befestigungselement 19 weist ein Innengewinde auf. Beispielsweise kann das Befestigungselement 19 als Mutter ausgebildet sein.

Die Vorrichtung weist ferner einen Zugkraftmesser 18 auf, über welchen die über das Zugkraftübertragungselement 15 übertragene Zugkraft gemessen werden kann. Der Zugkraftmesser 18 ist zwischen dem Halteelement 11 und dem auf dem Zugkraftübertragungselement 15 aufsitzenden Befestigungselement 19 angeordnet. Der Zugkraftmesser 18 weist eine Druckfeder 62 auf, welche ein erstes Federende umfasst, das gegenüber dem Halteelement 11 feststehend gelagert ist, und ein zweites Federende, das gegenüber dem auf dem Außengewinde des Zugkraftübertragungselements 15 aufsitzenden Befestigungselement 19 feststehend gelagert ist. Das erste Federende liegt an dem Halteelement 11 an und das zweite Federende liegt an dem Befestigungselement 19 an.

Der Zugkraftmesser 18 umfasst eine Skalenhülse 63 und eine Zeigerhülse 64, die konzentrisch zu dem Zugkraftübertragungselement 15 gegeneinander bewegbar angeordnet sind. Die Skalenhülse 63 weist mehrere, insbesondere drei, Bereiche auf, die mit unterschiedlichen Farben und/oder Schraffuren und/oder Symbolen versehen sind. Der Durchmesser der Zeigerhülse 64 ist größer als der Durchmesser der Skalenhülse 63, so dass die Zeigerhülse 64 die Skalenhülse 63 von außen umgreifen kann. Die Zeigerhülse 64 liegt an dem Befestigungselement 19 an und die Skalenhülse 63 an dem Haltelement 11.

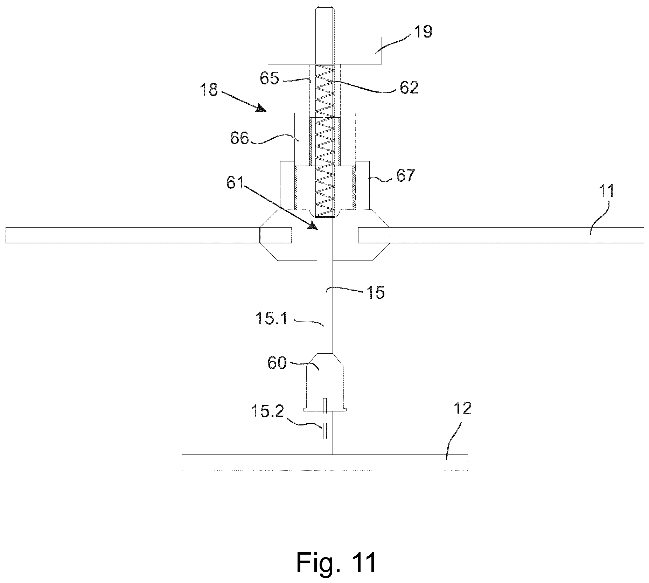

Die **Fig. 11** zeigt ein weiteres Ausführungsbeispiel einer erfindungsgemäßen Vorrichtung 10. Ebenso wie bei dem Ausführungsbeispiel nach Fig. 10 ist das Zugkraftübertragungselement 15 zweiteilig ausgebildet, wobei die beiden Teile 15.1, 15.2 des Zugkraftübertragungselements 15 über eine per Hand lösbare Kupplung 60 miteinander verbunden sind. Im Unterschied zu der in der Fig. 10 gezeigten Vorrichtung 10 weist der Zugkraftmesser 18 drei Hülsen 65, 66, 67 auf, welche konzentrisch zu dem Zugkraftübertragungselement 15 gegeneinander bewegbar angeordnet sind. Die Hülsen 65, 66, 67 sind mit unterschiedlichen Farben und/oder Schraffuren und/oder Symbolen versehen, so dass eine relative Anzeige der über das Zugkraftübertragungselement 15 übertragenen Zugkraft möglich ist.

Durch die in den Figuren dargestellte erfindungsgemäße Vorrichtung 10 gemäß der Ausführungsbeispiele kann die Faszie 4 derart weit offen gehalten werden, dass der Körperinnenraum, insbesondere der Bauchraum oder der innenliegend des Weichteildefekts liegende Raum, zugänglich ist und/oder die innenliegenden Organe durch den offenen Weichteildefekt, insbesondere die Bauchdeckenöffnung oder einen offenen Weichteildefekt am Rücken, zumindest im Wesentlichen ungehindert austreten können. Ferner kann die Vorrichtung 10 verwendet werden, um eine Faszie 4, deren Ränder sich bereits zurückgezogen haben, zu dehnen. Die erfindungsgemäße Vorrichtung kann optional verwendet werden, ohne dass eine Unterdruck-Wundtherapie durchgeführt wird.

### Bezugszeichenliste:

- 1: Patient
- 3: Haut
- 4: Faszie
- 10: Vorrichtung zur Verminderung der Retraktion der Faszie
- 11: Halteelement
- 12: Anbindungselement
- 12.1: Basisteil
- 12.2: Anbindungsbereich
- 12.3: Dichtbereich
- 13: Anbindungspunkte
- 14: Zugmittel
- 15: Zugkraftübertragungselement
- 16: Stütze
- 17: Dichtelement
- 18: Zugkraftmesser
- 19: Befestigungselement
- 20: Gurtbefestigung
- 21: Druckverteilungsmittel
- 25: Schwamm
- 26: Netz
- 27: Folie
- 28: Schwamm
- 30: Gelenk
- 31: Saugschlauch für Vakuumpumpe
- 40: Durchgangsbohrung
- 41: Sicherungselement
- 42: Verlustschutzelement
- 43: Führungshülse
- 44: Gewinde
- 45: Außengewinde
- 46: Einführnut
- 50: Verbindungsstelle
- 51: Sicherungsbolzen
- 52: Durchgangsbohrung
- 53: Anbindungspunkt
- 54: Abschnitt
- 55: Abschnitt
- 56: Federelement
- 57: Sackloch
- 60: Kupplung
- 61: Ausnehmung
- 62: Druckfeder
- 63: Skalenhülse
- 64: Zeigerhülse
- 65, 66, 67: Hülse

## Patentansprüche

1. Vorrichtung zur Verminderung der Retraktion der Ränder einer geöffneten Faszie eines Patienten mit einem offenen Weichteildefekt, wobei die Vorrichtung (10) ein Halteelement (11), welches in einem ersten Abstand von dem Patienten (1) gegenüber dem Patienten (1) festlegbar ist, und ein Anbindungselement (12) umfasst, welches eine Vielzahl von Anbindungspunkten (13) aufweist, die zur Anbindung von Zugmitteln (14) eingerichtet sind, die mit einem Rand der Faszie (4) verbindbar sind, wobei das Anbindungselement (12) mit dem Halteelement (11) derart verbunden ist, dass es in einem zweiten Abstand zu dem Patienten (1) angeordnet ist, welcher kleiner ist als der erste Abstand, wobei die Vorrichtung einen Zugkraftmesser (18) zur Messung der über ein Zugkraftübertragungselement (15) übertragenen Zugkraft aufweist,
**dadurch gekennzeichnet, dass** das Anbindungselement (12) mit dem Haltelement über genau ein Zugkraftübertragungselement verbunden ist.

2. Vorrichtung nach Anspruch 1, **dadurch gekennzeichnet, dass** das Zugkraftübertragungselement (15) in einer Ausnehmung (61) in dem Halteelement (11) geführt ist.

3. Vorrichtung nach einem der vorhergehenden Ansprüche, **dadurch gekennzeichnet, dass** das Zugkraftübertragungselement (15) einen Luftkanal aufweist.

4. Vorrichtung nach einem der vorhergehenden Ansprüche, **dadurch gekennzeichnet, dass** der Zugkraftmesser (18) als Federwaage ausgebildet ist.

5. Vorrichtung nach einem der vorhergehenden Ansprüche, **dadurch gekennzeichnet, dass** der Zugkraftmesser (18) eine Druckfeder aufweist, welche ein erstes Federende umfasst, das gegenüber dem Halteelement (11) feststehend gelagert ist, und ein zweites Federende, das gegenüber einem Mitnehmer des Zugkraftübertragungselements (15) oder gegenüber einem auf einem Außengewinde des Zugkraftübertragungselements (15) aufsitzenden Befestigungselement (19) feststehend gelagert ist.

6. Vorrichtung nach einem der vorhergehenden Ansprüche, **dadurch gekennzeichnet, dass** der Zugkraftmesser (18) eine Skalenhülse und eine Zeigerhülse aufweist, die konzentrisch zu dem Zugkraftübertragungselement (15) gegeneinander bewegbar angeordnet sind.

7. Vorrichtung nach einem der vorhergehenden Ansprüche, **dadurch gekennzeichnet, dass** der Zugkraftmesser (18) drei oder mehr Hülsen aufweist, welche konzentrisch zu dem Zugkraftübertragungselement (15) gegeneinander bewegbar angeordnet sind.

8. Vorrichtung nach einem der vorhergehenden Ansprüche, **dadurch gekennzeichnet, dass** das Zugkraftübertragungselement (15) starr ausgebildet ist.

9. Vorrichtung nach einem der vorhergehenden Ansprüche, **dadurch gekennzeichnet, dass** das Zugkraftübertragungselement (15) einen ersten Teil (15.1) und einen zweiten Teil (15.2) aufweist, welche wahlweise miteinander verbindbar oder voneinander trennbar sind, wobei bevorzugt zwischen dem ersten Teil (15.1) und dem zweiten Teil (15.2) eine lösbare Kupplung (60) angeordnet ist, die den ersten Teil (15.1) mit dem zweiten Teil (15.2) verbindet.

10. Vorrichtung nach einem der vorhergehenden Ansprüche, **dadurch gekennzeichnet, dass** das Zugkraftübertragungselement (15) mit dem Halteelement (11) und/oder mit dem Anbindungselement (12) über ein Gelenk (30, 32), insbesondere ein Kugelgelenk, verbunden ist.

11. Vorrichtung nach einem der vorhergehenden Ansprüche, **dadurch gekennzeichnet, dass** das Anbindungselement (12) zwei Anbindungsbereiche (12.2) aufweist, wobei die Anbindungsbereiche (12.2) jeweils mehrere Anbindungspunkte (13) aufweisen und ein Abstand zwischen den beiden Anbindungsbereichen (12.2) einstellbar ist; oder dass das Anbindungselement (12) zwei Anbindungsbereiche (12.2) aufweist, wobei die Anbindungsbereiche (12.2) jeweils mehrere Anbindungspunkte (13) aufweisen und die Anbindungsbereiche (12.2) gegeneinander bewegbar sind.

12. Vorrichtung nach Anspruch 11, **dadurch gekennzeichnet, dass** das Anbindungselement (12) ein Basisteil (12.1) aufweist und die Anbindungsbereiche (12.2) gegenüber dem Basisteil (12.1) bewegbar sind.

13. Vorrichtung nach einem der vorhergehenden Ansprüche, **dadurch gekennzeichnet, dass** die Vorrichtung (10) mehrere Stützen (16) aufweist, welche das Halteelement (11) jeweils mit einem auf die Haut des Patienten (1) auflegbaren Druckverteilungsmittel (21) verbinden; oder dass die Vorrichtung mehrere Stützen (16) aufweist, welche das Halteelement (11) mit einem Implantat oder mehreren Implantaten verbinden, welche an einem Knochen des Patienten (1) festlegbar sind.

14. Vorrichtung nach einem der vorhergehenden Ansprüche, **dadurch gekennzeichnet, dass** das Anbindungselement (12) im Bereich der Anbindungspunkte (13) jeweils eine Durchgangsbohrung (40) und eine in die Durchgangsbohrung (40) mündende Einführnut (46) aufweist, über welche ein Zugmittel (14) in die Durchgangsbohrung (40) einführbar ist.

15. Kit zur Behandlung eines offenen Weichteildefekts mit mindestens einem Schwamm (25) zum Ausfüllen des Weichteildefekts, **gekennzeichnet durch** eine Vorrichtung (10) nach einem der vorhergehenden Ansprüche.

## Claims

1. Device for reducing the retraction of the edges of an opened fascia of a patient having an open soft-tissue defect, the device (10) having a holding element (11), which can be fixed with respect to a patient (1) at a first distance from the patient (1), and an attachment element (12) which has a plurality of attachment points (13) configured for the attachment of tensioning means (14), which are connectable to an edge of the fascia (4), the attachment element (12) being connected to the holding element (11) so as to be arranged at a second distance from the patient (1) which is less than the first distance, the device having a tensile force gauge (18) for measuring the tensile force transmitted via a tensile force transmission element (15), **characterized in that** the attachment element (12) is connected to the holding element (11) via exactly one tensile force transmission element.

2. Device according to Claim 1, **characterized in that** the tensile force transmission element (15) is guided in a recess (61) in the holding element (11).

3. Device according to one of the preceding claims, **characterized in that** the tensile force transmission element (15) has an air duct.

4. Device according to one of the preceding claims, **characterized in that** the tensile force gauge (18) is designed as a spring balance.

5. Device according to one of the preceding claims, **characterized in that** the tensile force gauge (18) has a compression spring, which comprises a first spring end, the latter mounted to be stationary with respect to the holding element (11), and a second spring end, the latter mounted to be stationary with respect to a catch of the tensile force transmission element (15) or with respect to a fastening element (19) positioned on an external thread of the tensile force transmission element (15).

6. Device according to one of the preceding claims, **characterized in that** the tensile force gauge (18) has a scale sleeve and an indicator sleeve, which are arranged to be movable relative to each other concentrically with respect to the tensile force transmission element (15).

7. Device according to one of the preceding claims, **characterized in that** the tensile force gauge (18) has three or more sleeves, which are arranged to be movable relative to one another concentrically with respect to the tensile force transmission element (15).

8. Device according to one of the preceding claims, **characterized in that** the tensile force transmission element (15) is rigid.

9. Device according to one of the preceding claims, **characterized in that** the tensile force transmission element (15) has a first part (15.1) and a second part (15.2), which are selectively connectable to or separable from each other, wherein a detachable coupling (60) is preferably arranged between the first part (15.1) and the second part (15.2) and connects the first part (15.1) to the second part (15.2).

10. Device according to one of the preceding claims, **characterized in that** the tensile force transmission element (15) is connected to the holding element (11) and/or to the attachment element (12) via a joint (30, 32), in particular a ball joint.

11. Device according to one of the preceding claims, **characterized in that** the attachment element (12) has two attachment regions (12.2), wherein the attachment regions (12.2) each have a plurality of attachment points (13), and a distance between the two attachment regions (12.2) is adjustable; or **in that** the attachment element (12) has two attachment regions (12.2), wherein the attachment regions (12.2) each have a plurality of attachment points (13), and the attachment regions (12.2) are movable relative to each other.

12. Device according to Claim 11, **characterized in that** the attachment element (12) has a base part (12.1), and the attachment regions (12.2) are movable with respect to the base part (12.1).

13. Device according to one of the preceding claims, **characterized in that** the device (10) has a plurality of props (16) which each connect the holding element (11) to a pressure distribution means (21) that can be positioned on the skin of the patient (1); or **in that** the device has a plurality of props (16) which connect the holding element (11) to an implant or a plurality of implants that can be fixed to a bone of the patient (1).

14. Device according to one of the preceding claims, **characterized in that** the attachment element (12) has, in the region of the attachment points (13), a through-hole (40) and an insertion groove (46) opening into the through-hole (40), via which a tensioning means (14) is insertable into the through-hole (40).

15. Kit for treating an open soft-tissue defect, having at least one sponge (25) for filling out the soft-tissue defect, **characterized by** a device (10) according to one of the preceding claims.

## Revendications

1. Dispositif de réduction de la rétraction des bords d'un fascia ouvert d'un patient présentant un défaut de tissu mou ouvert, le dispositif (10) comprenant un élément de retenue (11), qui peut être fixé par rapport au patient (1) à une première distance du patient (1), et un élément de raccordement (12) qui comporte un grand nombre de points de raccordement (13) qui sont conçus pour raccorder des moyens de traction (14) qui peuvent être reliés à un bord du fascia (4), l'élément de raccordement (12) étant relié à l'élément de retenue (11) de manière à être disposé à une deuxième distance du patient (1) qui est inférieure à la première distance, le dispositif comportant un dynamomètre (18) destiné à mesurer la force de traction transmise par le biais d'un élément de transmission de force de traction (15), **caractérisé en ce que** l'élément de raccordement (12) est relié à l'élément de retenue par le biais d'exactement un élément de transmission de force de traction.

2. Dispositif selon la revendication 1, **caractérisé en ce que** l'élément de transmission de force de traction (15) est guidé dans un évidement (61) ménagé dans l'élément de retenue (11).

3. Dispositif selon l'une des revendications précédentes, **caractérisé en ce que** l'élément de transmission de force de traction (15) comporte un conduit d'air.

4. Dispositif selon l'une des revendications précédentes, **caractérisé en ce que** le dynamomètre (18) est conçu comme une balance à ressort.

5. Dispositif selon l'une des revendications précédentes, **caractérisé en ce que** le dynamomètre (18) comporte un ressort de compression qui comprend une première extrémité de ressort qui est montée de manière fixe par rapport à l'élément de retenue (11) et une deuxième extrémité de ressort qui est montée de manière fixe par rapport à un entraîneur de l'élément de transmission de force de traction (15) ou par rapport à un élément de fixation (19) siégeant sur un filetage extérieur de l'élément de transmission de force de traction (15).

6. Dispositif selon l'une des revendications précédentes, **caractérisé en ce que** le dynamomètre (18) comporte un manchon gradué et un manchon indicateur qui sont disposés de manière mobile l'un par rapport à l'autre concentriquement à l'élément de transmission de force de traction (15).

7. Dispositif selon l'une des revendications précédentes, **caractérisé en ce que** le dynamomètre (18) comporte trois manchons ou plus qui sont disposés de manière mobile l'un par rapport à l'autre concentriquement à l'élément de transmission de force de traction (15).

8. Dispositif selon l'une des revendications précédentes, **caractérisé en ce que** l'élément de transmission de force de traction (15) est rigide.

9. Dispositif selon l'une des revendications précédentes, **caractérisé en ce que** l'élément de transmission de force de traction (15) comporte une première partie (15.1) et une deuxième partie (15.2) qui peuvent au choix être reliées l'une à l'autre ou séparées l'une de l'autre, un accouplement amovible (60), qui relie la première partie (15.1) à la deuxième partie (15.2), étant de préférence disposé entre la première partie (15.1) et la deuxième partie (15.2).

10. Dispositif selon l'une des revendications précédentes, **caractérisé en ce que** l'élément de transmission de force de traction (15) est relié à l'élément de retenue (11) et/ou à l'élément de raccordement (12) par le biais d'une articulation (30, 32), notamment d'une rotule.

11. Dispositif selon l'une des revendications précédentes, **caractérisé en ce que** l'élément de raccordement (12) comporte deux zones de raccordement (12.2), les zones de raccordement (12.2) comportant chacune une pluralité de points de raccordement (13) et une distance entre les deux zones de raccordement (12.2) étant réglable ; ou **en ce que** l'élément de raccordement (12) comporte deux zones de raccordement (12.2), les zones de raccordement (12.2) comportant chacune une pluralité de points de raccordement (13) et les zones de raccordement (12.2) pouvant être déplacées les unes par rapport aux autres.

12. Dispositif selon la revendication 11, **caractérisé en ce que** l'élément de raccordement (12) comporte une partie de base (12.1) et les zones de raccordement (12.2) sont mobiles par rapport à la partie de base (12.1).

13. Dispositif selon l'une des revendications précédentes, **caractérisé en ce que** le dispositif (10) comporte une pluralité de supports (16) qui relient chacun l'élément de retenue (11) à un moyen de répartition de pression (21) pouvant être placé sur la peau du patient (1) ; ou **en ce que** le dispositif comporte une pluralité de supports (16) qui relient l'élément de retenue (11) à un implant ou à une pluralité d'implants qui peuvent être fixés à un os du patient (1).

14. Dispositif selon l'une des revendications précédentes, **caractérisé en ce que** l'élément de raccordement (12) comporte au niveau de chacun des points de raccordement (13) un trou traversant (40) et une rainure d'insertion (46) qui débouche dans le trou traversant (40) et qui permet d'insérer un moyen de traction (14) dans le trou traversant (40).

15. Kit de traitement d'un défaut de tissu mou ouvert, ledit kit comprenant au moins une éponge (25) destinée à combler le défaut de tissu mou, **caractérisé par** un dispositif (10) selon l'une des revendications précédentes.
